# EUROPEAN PATENT APPLICATION

(11) **EP 4 104 783 A1**
(43) Date of publication of application: **21.12.2022**
(21) Application number: 22178859.9
(22) Date of filing: 14.06.2022
(51) Int. Cl.: A61B 18/14

(54) **CATHETER FOR HIGH-POWER FOCAL ABLATION**

(30) Priority: 15.06.2021 US 202117347986
(71) Applicant: Biosense Webster (Israel) Ltd, Yokneam, 2066717 (IL)
(72) Inventor: GOVARI, Assaf, 2066717 Yokneam (IL); BEECKLER, Christopher Thomas, Irvine, 92618 (US); KEYES, Joseph Thomas, Irvine, 92618 (US)
(74) Representative: Carpmaels & Ransford LLP

(57) **Abstract**

Medical apparatus includes a flexible insertion tube, which has a first outer diameter and has a distal end configured for insertion into a cavity within a body of a patient. A rigid cylindrical electrode is fixed to the distal end of the flexible insertion tube and is configured to contact tissue within the cavity, and which has a second outer diameter that is at least 10% greater than the first outer diameter.

## Description

### FIELD OF THE INVENTION

The present invention relates generally to invasive medical devices, and particularly to ablation catheters and methods for their use.

### BACKGROUND

Radiofrequency (RF) ablation of the heart is widely used to correct cardiac arrhythmias, such as atrial fibrillation. The ablation procedure typically involves insertion of a catheter, having one or more electrodes at its distal end, into the heart, and application of RF energy to the electrode or electrodes in order to ablate selected regions within the heart. Various sorts of catheters can be used for this purpose. As one example, the QDOT MICRO^{®} catheter, produced by Biosense Webster (Irvine, California), enables ablation at up to 90 watts of RF power for up to four seconds in a temperature-controlled ablation mode.

A catheter of this sort is described in U.S. Patent 10,517,667, whose disclosure is incorporated herein by reference. The catheter comprises an insertion tube, which has a distal tip that is configured to act as an electrode for the ablation. The distal tip, typically formed in a generally cup-like shape, has at least one cavity formed in its external surface. For each cavity there is a respective microelectrode configured to fit into the cavity, with an outer surface contoured to conform with the outer surface of the distal tip. Typically the microelectrode is insulated from the distal tip so that the microelectrode is able to detect electrical potentials generated by the body with high spatial resolution, independent of far-field signals picked up by the larger distal tip.

High-power focal ablation catheters may include means for irrigating the area of the electrode. For example, U.S. Patent Application Publication 2018/0104000, whose disclosure is incorporated herein by reference, describes a medical probe having at least one distal electrode coupled to an energy source to apply energy to tissue inside the body. A plurality of apertures are formed in the electrode. A fluid-directing assembly, disposed in the distal section of the probe, has an axial channel that is in fluid communication with at least one trans-axial channel disposed at right angles to the axial channel and leading to an exterior of the assembly. A blocking terminus, disposed forward of the at least one trans-axial channel, prevents irrigation fluid from flowing axially in a forward direction so that the fluid flows outwardly into the lumen of the electrode in at least one trans-axial direction and exits the electrode through the apertures.

The distal end of an ablation catheter may also include sensors of other types. For example, U.S. Patent Application Publication 2019/0117298, whose disclosure is incorporated herein by reference, describes a medical probe, consisting of an insertion tube having a distal end configured for insertion into a body of a patient and containing a lumen having an electrical conductor for conveying electrical energy. The probe also has a conductive cap attached to the distal end of the insertion tube and coupled electrically to the electrical conductor. The cap includes a side wall having multiple longitudinal bores therein. A plurality of thermocouples are disposed in the longitudinal bores, and an electrically conductive cement at least partially fills the longitudinal bores so as to secure the thermocouples in the bores while making electrically conductive contact between the thermocouples and the conductive cap.

### SUMMARY

Embodiments of the present invention that are described hereinbelow provide improved catheters and methods for their fabrication and use.

There is therefore provided, in accordance with an embodiment of the invention, medical apparatus, including a flexible insertion tube, which has a first outer diameter and has a distal end configured for insertion into a cavity within a body of a patient. A rigid cylindrical electrode is fixed to the distal end of the flexible insertion tube and is configured to contact tissue within the cavity, and which has a second outer diameter that is at least 10% greater than the first outer diameter.

In a disclosed embodiment, the second diameter is at least 20% greater than the first outer diameter. Additionally or alternatively, the second outer diameter is at least 3 mm. Further additionally or alternatively, the rigid cylindrical electrode has a rounded distal edge.

In some embodiments, the apparatus includes an electrical conductor passing through the flexible insertion tube and configured to apply radiofrequency (RF) electrical energy to the rigid cylindrical electrode with a power sufficient to ablate the tissue. In a disclosed embodiment, the power of the RF electrical energy applied to the electrode by the electrical conductor is at least 100 W.

Additionally or alternatively, the rigid cylindrical electrode includes an exterior wall containing a central volume and has apertures formed through the exterior wall in communication with the central volume, and the apparatus includes a lumen passing through the flexible insertion tube and coupled to supply a fluid to the central volume, whereby the fluid exits through the apertures to irrigate the tissue. In a disclosed embodiment, the lumen is configured to input the fluid to the central volume along an axial direction, and the apparatus includes a flow diverter, which is disposed within the central volume of the cylindrical electrode and is configured to deflect the fluid in a radial direction toward the apertures.

In some embodiments, the rigid cylindrical electrode includes a distal face and a side face with a circular edge between the distal and side faces, and the distal face contains a plurality of elongated grooves, which extend radially outward at different azimuthal angles across a peripheral area of the distal face through the edge, and the apparatus includes multiple sensors, which are disposed within the elongated grooves so as to contact the tissue that is contacted by the rigid cylindrical electrode. In one embodiment, the sensors include sensing electrodes, which are configured to sense electrophysiological signals generated by the tissue with which the sensing electrodes are in contact. Additionally or alternatively, the circular edge has a rounded profile extending between the distal and side faces, and the sensors are sized and shaped to fit within the elongated grooves such that an outer surface of each of the sensors is flush with the distal face and with the rounded profile of the circular edge.

There is also provided, in accordance with an embodiment of the invention, a method of treatment, which includes inserting a catheter into a chamber of a heart of a patient and bringing an electrode at a distal end of the catheter into contact with tissue within the heart. Radiofrequency (RF) electrical energy is applied through the catheter to the electrode with a power of at least 100 W so as to ablate the tissue.

The present invention will be more fully understood from the following detailed description of the embodiments thereof, taken together with the drawings in which:

### BRIEF DESCRIPTION OF THE DRAWINGS

Fig. 1 is a schematic side view of the distal end of an ablation catheter, in accordance with an embodiment of the invention; and
Fig. 2 is a schematic sectional view of the distal tip of the catheter of Fig. 1, in accordance with an embodiment of the invention.

### DETAILED DESCRIPTION OF EMBODIMENTS

High-power focal ablation, using devices such as the above-mentioned QDOT MICRO catheter, has been found to be therapeutically advantageous, particularly since this approach shortens significantly the time needed to perform the procedure. The term "focal ablation," as used in the present description, refers to application of energy to tissue in the body, such as myocardial tissue, at a single location. Focal ablation typically uses a single electrode at the tip of a catheter or other probe, rather than ablating over a range of locations simultaneously using a probe with a multi-electrode distal assembly, such as a basket, balloon, or lasso assembly. Using the QDOT MICRO catheter with RF power of 90 W, focal ablation at a single location can be accomplished in as little as 4 sec.

It would be desirable to have the capability to create deeper lesions, as well as shorten the ablation time even further, using a higher level of RF power. Shorter ablation time also reduces the likelihood that the ablation electrode might shift or lose contact with the myocardium during the ablation. Power delivery in focal catheters that are known in the art, however, is also limited by the diameter of the catheter tip electrode, since excessive power may cause overheating and damage to the catheter tip and body tissue. The electrode diameter, in turn, has up to now been limited to the diameter of the catheter insertion tube, and lesion depth in focal catheters that are known in the art is limited to approximately three to four times the tip diameter due to the biophysics of RF ablation. The insertion tube diameter is typically in the range of 2.5 mm to enable the catheter to be inserted easily through the patient's vascular system into the heart. Therefore, the RF ablation power has been practically limited to levels below 100 W, and lesion depths have been limited to less than 10 mm.

Embodiments of the present invention that are described herein overcome these limitations by providing apparatus, such as a cardiac catheter, in which a rigid cylindrical electrode has an outer diameter that is at least 10% greater than that of the flexible insertion tube to whose distal end the electrode is fixed. In some embodiments, the electrode diameter is at least 20% greater than the insertion tube diameter. In typical cardiac ablation applications, this criterion means that the outer diameter of the rigid cylindrical electrode is 3 mm or more. By virtue of these increased dimensions, the volume of the tip electrode can be made 50-100% greater than that of catheters that are known in the art and thus will support the application of RF ablation power in excess of 100 W, as well as creating ablation lesions of greater depth. Given the moderate increase in electrode size, however, it is still possible to maneuver the catheter atraumatically through the patient's vascular system into the heart.

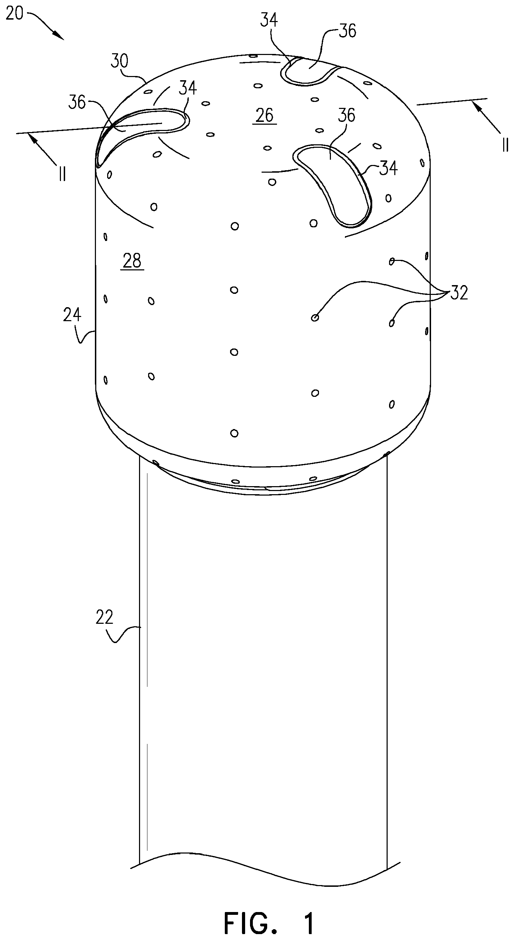

Fig. 1 is a schematic side view of the distal end of an ablation catheter 20, in accordance with an embodiment of the invention. Catheter 20 comprises a flexible insertion tube 22, whose distal end is configured for insertion into a cavity within a body of a patient, such as a chamber of the heart. A rigid cylindrical electrode 24 is fixed to the distal end of insertion tube 22 and is configured to contact tissue within the cavity. The outer diameter of electrode 24 in the pictured example is about 3.7 mm, and is more than 20% greater than the outer diameter of insertion tube 22, which is less than 3 mm.

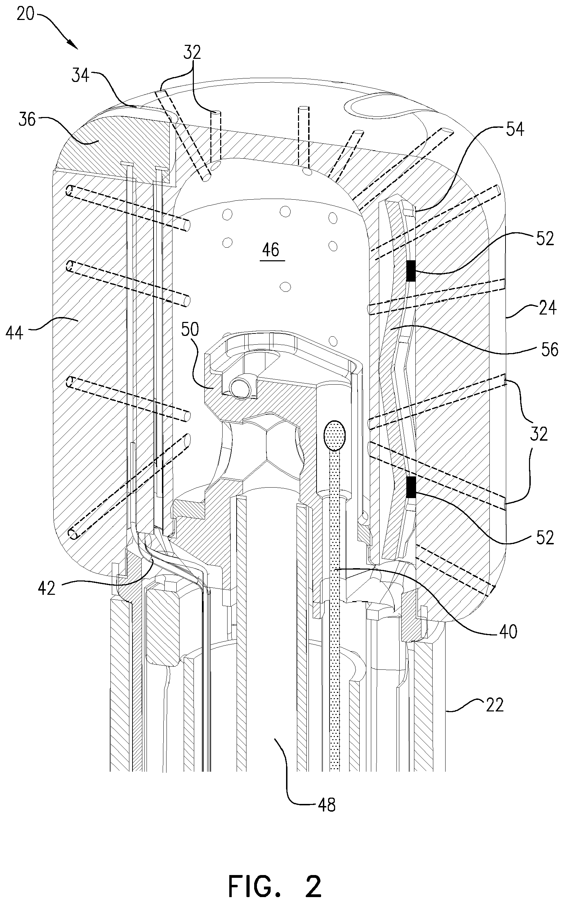

Electrode 24 comprises a suitable biocompatible metal, such as gold. The electrode is formed as a cylinder with rounded edges, comprising a planar distal face 26 and a round side face 28, with a circular edge 30 between faces 26 and 28. Edge 30 has a rounded profile, for example similar to that of the distal tip of the catheter that is described in the above-mentioned U.S. Patent 10,517,667. Irrigation apertures 32 pass through the outer shell of electrode 24 and enable irrigation fluid, such as saline solution, to exit through the apertures and irrigate the tissue with which the electrode is in contact. Details of this irrigation mechanism are shown in Fig. 2.

Distal face 26 of electrode 24 contains a plurality of elongated grooves 34, which extend radially outward at different azimuthal angles across the peripheral area of the distal face and pass through edge 30 to side face 28. (The "azimuthal angle" refers to the angle of rotation about the longitudinal axis of catheter 20, and radial directions are perpendicular to this axis, in the plane of distal face 26. The "peripheral area" refers to the area in proximity to edge 30, as opposed to the central area in proximity to the longitudinal axis. The grooves are "elongated" in the sense that their length in the radial direction is at least twice their width in the azimuthal direction.) In the present example, electrode 24 contains three grooves 34 situated 120° apart; but alternatively, the electrode may contain a larger or smaller number of such grooves.#

Sensing electrodes 36 are fitted into elongated grooves 34 but are electrically insulated from the metal body of electrode 24. Sensing electrodes 36 contact the tissue that is contacted by electrode 24 and thus sense electrophysiological signals generated by the tissue with which the sensing electrodes are in contact. These signals provide an indication of local electrical activity in the tissue before, after, and even during the ablation procedure. Alternatively or additionally, other types of sensors, such as thermocouples, may be fitted into grooves 34, as well as in other locations within the body of electrode 24. Sensing electrodes 36 are themselves elongated and are sized and shaped to fit within grooves 34 such that the outer surface of each sensing electrode 36 is flush with distal face 26 and with the rounded profile of circular edge 30. This flush configuration is useful in creating an atraumatic tip and ensuring uniform electrical contact and avoiding electrical hot spots and arcing.

Catheter 20 can be used for intracardiac ablation, for example in place of the catheters described in the above-mentioned U.S. Patent 10,517,667 and U.S. Patent Application Publications 2018/0104000 and 2019/0117298, but at an increased RF power level. When catheter 20 is inserted into a chamber of the heart of a patient, and electrode 24 is brought into contact with tissue within the heart, RF electrical energy can be applied through the catheter to the electrode with a power of at least 100 W so as to ablate the tissue. The systems described in U.S. Patent 10,517,667 and U.S. Patent Application Publications 2018/0104000 and 2019/0117298 include RF generators, irrigation pumps, and sensing circuits, which may likewise be applied, *mutatis mutandis,* to drive, irrigate, and receive signals from catheter 20. U.S. Patent Application Publications 2018/0104000 and 2019/0117298 also describe temperature sensors, contact force sensors, and position sensors that can likewise be integrated into the distal end of catheter 20; but these additional sensors are omitted from the present description for the sake of brevity.

Fig. 2 is a schematic sectional view of the distal tip of catheter 20, taken along the line II-II in Fig. 1, in accordance with an embodiment of the invention. As can be seen in this figure, electrode 24 is connected to receive RF ablation energy via an electrical conductor 40 passing through insertion tube 22 to a control console, as shown in the above-mentioned U.S. patent and patent applications. (In the pictured embodiment, for example, conductor 40 is electrically and mechanically secured by soldering it into the cylindrical distal space in flow diverter 50.) As noted earlier, conductor 40 is capable of conveying RF energy to electrode 24 with a power of at least 100 W, which is sufficient to rapidly ablate tissue contacted by the electrode. Sensing electrodes 36 are likewise connected by wires 42 passing through insertion tube 22 to sensing circuits in the console.

Rigid cylindrical electrode 24 comprises an exterior wall 44, which encloses and contains a central volume 46. A lumen 48 passing through flexible insertion tube 22 supplies a fluid to central volume 46 along the axial direction. The fluid exits central volume 46 through apertures 32 to irrigate the tissue in proximity to electrode 24. A flow diverter 50 within central volume 46 deflects the fluid entering through lumen 48 in a radial direction toward apertures 32, to ensure even dispersal of the fluid to the entire area surrounding electrode 24. Apertures 32 themselves are angled to enhance the fluid dispersal. Further features of this arrangement for tissue irrigation are described in the above-mentioned U.S. Patent Application Publication 2018/0104000.

In some embodiments, catheter 20 also has temperature sensing capabilities. For this purpose, temperature sensors 52, such as thermocouples, are inserted into bores 54 within electrode 24. A resilient curved rod 56 holds temperature sensors 52 in place, with the temperature sensors facing toward the outer surface of electrode 24, to ensure reliable measurements. In the embodiment shown in Fig. 2, each bore 54 contains two temperature sensors 52 in different axial locations.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

Aspects of the invention:
1. A method of treatment, comprising:
   inserting a catheter into a chamber of a heart of a patient and bringing an electrode at a distal end of the catheter into contact with tissue within the heart; and
   applying radiofrequency (RF) electrical energy through the catheter to the electrode with a power of at least 100 W so as to ablate the tissue.
2. The method according to aspect 1, wherein the catheter comprises a flexible insertion tube, which has a first outer diameter, and wherein the electrode comprises a rigid cylindrical electrode, which is fixed to a distal end of the flexible insertion tube has a second outer diameter that is at least 10% greater than the first outer diameter.
3. The method according to aspect 2, wherein the second diameter is at least 20% greater than the first outer diameter.
4. The method according to aspect 2, wherein the second outer diameter is at least 3 mm.
5. The method according to aspect 2, wherein the rigid cylindrical electrode has a rounded distal edge.
6. The method according to aspect 1, and comprising supplying a fluid through apertures in the electrode to irrigate the tissue.
7. The method according to aspect 6, wherein supplying the fluid comprises inputting the fluid through a lumen in the catheter to a central volume of the electrode along an axial direction, and applying a flow diverter within the central volume to deflect the fluid in a radial direction toward the apertures.
8. The method according to aspect 1, and comprising sensing electrophysiological signals generated by the tissue using a plurality of elongated sensors, which extend radially outward at different azimuthal angles across a peripheral area of the electrode.

## Claims

1. Medical apparatus, comprising:
a flexible insertion tube, which has a first outer diameter and has a distal end configured for insertion into a cavity within a body of a patient; and
a rigid cylindrical electrode, which is fixed to the distal end of the flexible insertion tube and is configured to contact tissue within the cavity, and which has a second outer diameter that is at least 10% greater than the first outer diameter.

2. The apparatus according to claim 1, wherein the second diameter is at least 20% greater than the first outer diameter.

3. The apparatus according to claim 1, wherein the second outer diameter is at least 3 mm.

4. The apparatus according to claim 1, wherein the rigid cylindrical electrode has a rounded distal edge.

5. The apparatus according to claim 1, and comprising an electrical conductor passing through the flexible insertion tube and configured to apply radiofrequency (RF) electrical energy to the rigid cylindrical electrode with a power sufficient to ablate the tissue.

6. The apparatus according to claim 5, wherein the power of the RF electrical energy applied to the electrode by the electrical conductor is at least 100 W.

7. The apparatus according to claim 1, wherein the rigid cylindrical electrode comprises an exterior wall containing a central volume and has apertures formed through the exterior wall in communication with the central volume, and wherein the apparatus comprises a lumen passing through the flexible insertion tube and coupled to supply a fluid to the central volume, whereby the fluid exits through the apertures to irrigate the tissue.

8. The apparatus according to claim 7, wherein the lumen is configured to input the fluid to the central volume along an axial direction, and the apparatus comprises a flow diverter, which is disposed within the central volume of the cylindrical electrode and is configured to deflect the fluid in a radial direction toward the apertures.

9. The apparatus according to claim 1, wherein the rigid cylindrical electrode comprises a distal face and a side face with a circular edge between the distal and side faces, and the distal face contains a plurality of elongated grooves, which extend radially outward at different azimuthal angles across a peripheral area of the distal face through the edge, and
wherein the apparatus comprises multiple sensors, which are disposed within the elongated grooves so as to contact the tissue that is contacted by the rigid cylindrical electrode.

10. The apparatus according to claim 9, wherein the sensors comprise sensing electrodes, which are configured to sense electrophysiological signals generated by the tissue with which the sensing electrodes are in contact.

11. The apparatus according to claim 9, wherein the circular edge has a rounded profile extending between the distal and side faces, and wherein the sensors are sized and shaped to fit within the elongated grooves such that an outer surface of each of the sensors is flush with the distal face and with the rounded profile of the circular edge.
